# EUROPEAN PATENT APPLICATION

(11) **EP 4 360 878 A1**
(43) Date of publication of application: **01.05.2024**
(21) Application number: 22849528.9
(22) Date of filing: 27.07.2022
(51) Int. Cl.: B32B 37/24, A61F 13/15

(54) **MANUFACTURING DEVICE AND MANUFACTURING METHOD FOR ELASTIC LAMINATE**

(30) Priority: 27.07.2021 JP 2021122533
(71) Applicant: Zuiko Corporation, Ibaraki-shi Osaka 5670082 (JP)
(72) Inventor: FUJITA, Yukihiko, Ibaraki-shi, Osaka 567-0082 (JP); MINAMI, Asuka, Ibaraki-shi, Osaka 567-0082 (JP)
(74) Representative: Hasegawa, Kan
(86) International application number: PCT/JP2022/028893
(87) International publication number: WO 2023/008467

(57) **Abstract**

A manufacturing device (1) includes a guiding part (4) that guides an elastic member (EL) between a pair of sheets (S1, S2) conveyed by a pair of nip rolls (21); a moving mechanism (5) that reciprocates the guiding part (4) along a moving path (MP); and a reciprocation control part (7a). The moving mechanism (5) has a first rotation member (50) that is rotatable about a first rotation center shaft (50a) and capable of changing a first rotation radius connecting the guiding part (4) and the first rotation center shaft (50a) to each other, and a second rotation member (55) that is rotatable about a second rotation center shaft (55a), connected to the first rotation member (50) so as to be rotatable each other, and capable of changing a second rotation radius connecting the guiding part (4) and the second rotation center shaft (55a) to each other. The reciprocation control part (7a) synchronizes rotation of the first rotation member (50) and rotation of the second rotation member (55) with each other.

## Description

### Technical Field

The present invention relates to a manufacturing device and a manufacturing method for an elastic laminate.

### Background Art

An elastic laminate including a pair of sheets and an elastic member nipped between the pair of sheets is conventionally known as a material constituting a paper diaper, for example. As a device for manufacturing such an elastic laminate, for example, Patent Literature 1 discloses a device including a pair of nip rolls and a drive mechanism provided with a guiding part. The pair of nip rolls nips the pair of sheets in a state where the elastic member is interposed between the pair of sheets. The guiding part is provided upstream of the pair of nip rolls in a conveying direction of the pair of sheets, and guides the elastic member between the pair of sheets. The drive mechanism reciprocates the guiding part along a preset moving path so as to arrange the elastic member along a preset path with respect to the pair of sheets.

The drive mechanism includes a moving part for reciprocating the guiding part, and a restriction mechanism that allows the guiding part to move along the moving path and restricts movement in a direction orthogonal to the moving path. The moving part includes a belt and a pair of pulleys for bridging the belt along the moving path. The restriction mechanism includes the slider attached to the belt, a rail that supports the slider so as to be slidable along the moving path, and the guiding part attached to the belt. The belt reciprocates by the pair of pulleys sequentially rotating in forward and reverse directions. Due to this, the slider slides along the rail, whereby the guiding part reciprocates along the moving path.

Slide of the slider along the rail is accompanied by wear of the slider and the rail. As the friction progresses, restriction of movement of the guiding part by the restriction mechanism may be weakened, and the guiding part may deviate from the moving path. A large load acts on the slider due to high-speed reciprocation of the slider with respect to the rail, which may facilitate deterioration of the restriction mechanism. In order to suppress such a situation, it is necessary to perform maintenance including inspection, replacement, and repair of the restriction mechanism at a high frequency.

Furthermore, when the restriction mechanism is used, there is a limit to an increase in the manufacturing speed of the elastic laminate. Specifically, since sliding resistance is generated between the slider and the rail when the slider reciprocates, there is a limit to an increase in the speed at which the slider moves on the rail. Therefore, it is desired to omit the restriction mechanism.

### Citation List

### Patent Literature

Patent Literature 1: Japanese Patent No. 6423798

### Summary of Invention

The present invention has been made in view of the above circumstances, and an object is to provide a manufacturing device for an elastic laminate capable of reciprocating the guiding part along the moving path without using a restriction mechanism accompanied by sliding friction of a slider with respect to a rail and a manufacturing method for the elastic laminate.

A manufacturing device for an elastic laminate for solving the above problems provides a device for manufacturing an elastic laminate including a pair of sheets extending in a longitudinal direction and an elastic member nipped between the pair of sheets, the manufacturing device for an elastic laminate, including: a pair of nip rolls arranged in a state where each axis is orthogonal to the longitudinal direction so as to convey the pair of sheets and the elastic member in a conveying direction parallel to the longitudinal direction while nipping the pair of sheets in a state where the elastic member is interposed between the pair of sheets; a feeding mechanism including a guiding part that is provided upstream of the pair of nip rolls in the conveying direction and guides the elastic member between the pair of sheets while feeding the elastic member such that a longitudinal direction of the elastic member is along a feeding direction orthogonal to the axis, and a moving mechanism capable of reciprocating the guiding part in a moving direction parallel to the axis; and a reciprocation control part that controls the moving mechanism so that the guiding part reciprocates along a moving path extending in the moving direction, in which the moving mechanism includes a first rotation member rotatable about a first rotation center shaft orthogonal to a moving path plane including the moving path at a position away from the guiding part in the moving path plane and configured such that a first rotation radius connecting the guiding part and the first rotation center shaft is changeable, a first rotation drive part that rotates the first rotation member about the first rotation center shaft, a second rotation member rotatable about a second rotation center shaft parallel to the first rotation center shaft at a position away from the guiding part in the moving path plane, connected to the first rotation member so as to be rotatable each other, and configured such that a second rotation radius connecting the guiding part and the second rotation center shaft is changeable, and a second rotation drive part that rotates the second rotation member around the second rotation center shaft, and the reciprocation control part performs reciprocation control of synchronizing rotation of the first rotation member by the first rotation drive part and rotation of the second rotation member by the second rotation drive part so as to reciprocate the guiding part along the moving path.

A manufacturing method for an elastic laminate for solving the above problems provides a method for manufacturing an elastic laminate using the manufacturing device for an elastic laminate, the manufacturing method for an elastic laminate, including: a process of preparing the manufacturing device for the elastic laminate; a process of conveying the pair of sheets in the conveying direction so as to be guided between the pair of nip rolls; a process of synchronizing rotation of the first rotation member by the first rotation drive part and rotation of the second rotation member by the second rotation drive part so as to reciprocate the guiding part along the moving path; and a process of guiding the elastic member between the pair of sheets while feeding the elastic member in a longitudinal direction of the elastic member using the guiding part such that the elastic member is nipped between the pair of sheets by the pair of nip rolls.

According to the manufacturing device for the elastic laminate and the manufacturing method for the elastic laminate of the present invention, it is possible to reciprocate the guiding part along the moving path without using a restriction mechanism accompanied by sliding friction of a slider with respect to a rail.

### Brief Description of Drawings

FIG. 1 is a schematic view illustrating a manufacturing process of an elastic laminate manufactured by a manufacturing device according to an embodiment of the present invention.
FIG. 2 is a front view illustrating an overall configuration of the manufacturing device according to the embodiment of the present invention.
FIG. 3 is a rear view illustrating an overall configuration of the manufacturing device according to the embodiment of the present invention.
FIG. 4 is a cross-sectional view taken along line IV-IV in FIGS. 2 and 3, and is an elastic member arrangement part for a back waistline part.
FIG. 5 is a cross-sectional view taken along line V-V in FIGS. 2 and 3, and is an elastic member arrangement part for a front waistline part.
FIG. 6 is an enlarged view of a guiding part.
FIG. 7 is a plan view illustrating a situation of retraction control.
FIG. 8 is a flowchart showing processing executed by a control part.
FIG. 9 is a chart illustrating a movement pattern of the guiding part set for each size.
FIG. 10 is a chart illustrating a drive pattern of a first rotation drive part set based on the chart of FIG. 9.
FIG. 11 is a chart illustrating a drive pattern of a second rotation drive part set based on the chart of FIG. 9.
FIG. 12 is a front view illustrating an overall configuration of a manufacturing device in a form different from the embodiment.
FIG. 13 is a cross-sectional view taken along line IX-IX in FIG. 12.
FIG. 14 is a front view illustrating an overall configuration of a manufacturing device in a yet different from the embodiment.
FIG. 15 is a cross-sectional view taken along line XI-XI in FIG. 14.

### Description of Embodiments

An embodiment of the present invention will be described below with reference to the accompanying drawings. Note that the following embodiment is an example embodying the present invention, and is not intended to limit the technical scope of the present invention.

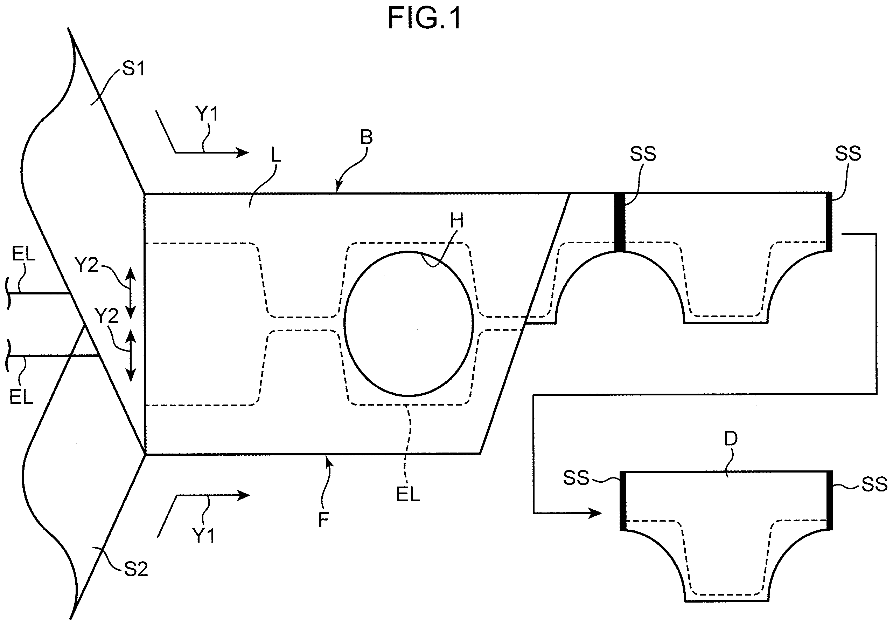

First, an elastic laminate L manufactured by a manufacturing device according to an embodiment of the present invention will be described with reference to FIG. 1.

The elastic laminate L includes a pair of sheets S1 and S2 extending in a sheet longitudinal direction and a plurality of elastic members EL nipped between the pair of sheets S1 and S2. The plurality of elastic members EL are arranged along a path meandering with respect to the sheet longitudinal direction in the pair of sheets S1 and S2. The sheet width direction is orthogonal to the sheet longitudinal direction. In the example of FIG. 1, the plurality of elastic members EL include a plurality of upper elastic members EL arranged on an upper side with respect to a center part in the sheet width direction of the pair of sheets S1 and S2, and a plurality of lower elastic members EL arranged on a lower side. The plurality of elastic members EL are arranged between the pair of sheets S1 and S2 so that a peak part and a bottom part of a substantially sine curve in the sheet longitudinal direction defined by the plurality of upper elastic members EL and respective bottom part and peak part of a substantially sine curve in the sheet longitudinal direction defined by the plurality of lower elastic members EL have the same phase.

Such the elastic laminate L is used for a disposable diaper D, for example. Specifically, in FIG. 1, an upper side of the elastic laminate L with respect to a substantially center part in the sheet width direction is a front waistline part F covering an anterior abdomen part, and a lower side thereof is a back waistline part B covering a back part. A leg hole H is formed at a position where the plurality of upper elastic members EL and the plurality of lower elastic members EL in each of the front waistline part F and the back waistline part B are farthest from each other. The disposable diaper D includes a side seal part SS in which parts of the front waistline part F and the back waistline part B corresponding to a part from an outer side of a root of a wearer's leg to a waist are welded in a state where the front waistline part F and the back waistline part B are overlapped with each other.

The elastic laminate L is manufactured as follows. First, an adhesive is applied to at least one of the pair of sheets S1 and S2 and the plurality of elastic members EL.

Next, as indicated by an arrow Y1, the pair of sheets S1 and S2 is conveyed in a conveying direction parallel to the sheet longitudinal direction so as to merge at a preset merging position.

Together with conveyance of the pair of sheets S1 and S2, the plurality of elastic members EL are guided to a position between the pair of sheets S1 and S2 at the merging position while feeding the plurality of elastic members EL in a longitudinal direction thereof. In the example of FIG. 1, as indicated by an arrow Y2, each of the plurality of elastic members EL is guided between the pair of sheets S1 and S2 while reciprocating in the sheet width direction of the pair of sheets S1 and S2.

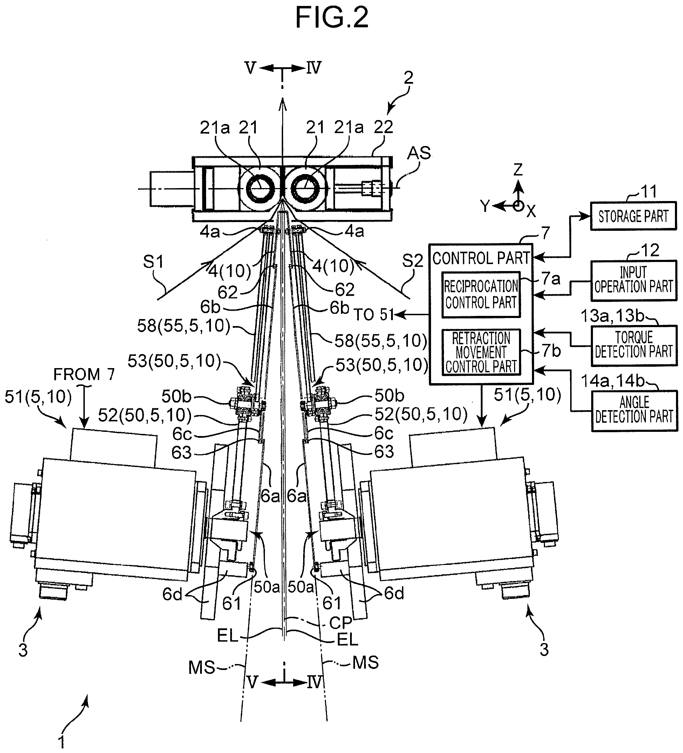

At the merging position, the pair of sheets S1 and S2 and the plurality of elastic members EL in a state of being changed in position in the sheet width direction are nipped by a pair of nip rolls 21 (see FIG. 2) described later. Due to this, the pair of sheets S1 and S2 and the plurality of elastic members EL are bonded to each other, and the elastic laminate L is manufactured.

In a case of manufacturing the disposable diaper D, the leg hole H is formed in the elastic laminate L. The elastic laminate L is folded in half in the sheet width direction so that the front waistline part F and the back waistline part B overlap each other.

Next, the elastic laminates L folded in half are welded in the sheet width direction at a part near the apex of the leg hole H to form the side seal part SS, and the elastic laminate L is cut so that the side seal part SS remains on both sides to cut the elastic laminate L into the disposable diaper D.

Note that an absorber can be joined to a position between the adjacent leg holes H in the elastic laminate L before being folded in half.

Hereinafter, a manufacturing device 1 for the elastic laminate L will be described with reference to FIGS. 2 to 5.

The manufacturing device 1 includes a nipping part 2 including the pair of nip rolls 21 for conveying the pair of sheets S1 and S2, and a pair of elastic member arrangement parts 3 for guiding the upper elastic member EL and the lower elastic member EL between the pair of sheets S1 and S2, respectively.

The pair of nip rolls 21 conveys the pair of sheets S1 and S2 and the elastic member EL in the conveying direction while nipping the pair of sheets S1 and S2 in a state where the plurality of elastic members EL are interposed between the pair of sheets S1 and S2. An axis 21a of each of the pair of nip rolls 21 extends in parallel with the sheet width direction. The pair of nip rolls 21 is arranged in a state where each axis 21a is orthogonal to the sheet longitudinal direction.

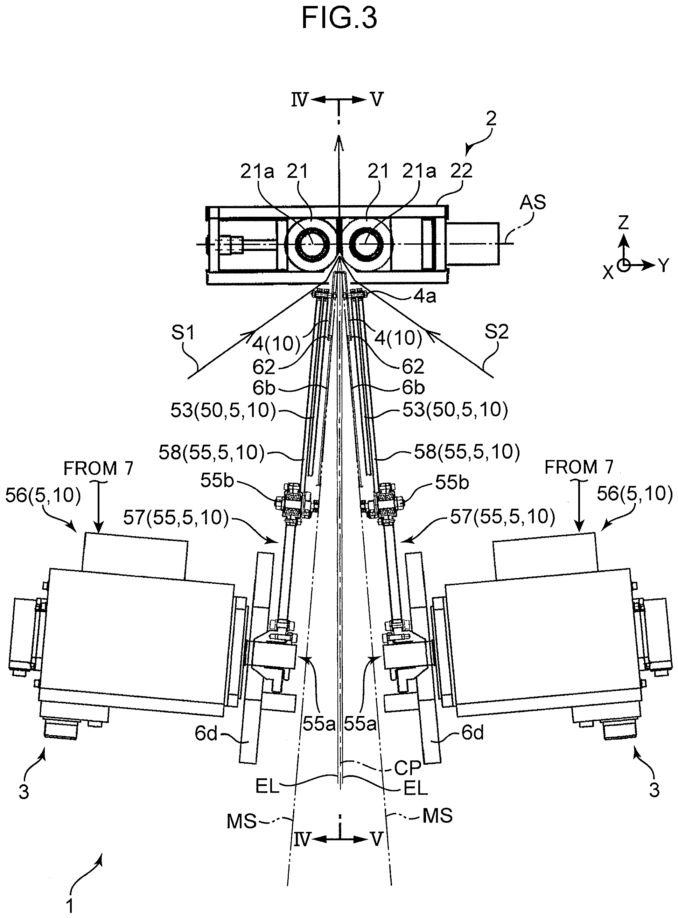

In the following description, the direction along the axis 21a of each of the pair of nip rolls 21 is an X direction in FIGS. 2 to 5. A direction orthogonal to the axis 21a of each of the pair of nip rolls 21 and a direction in which the pair of nip rolls 21 is arranged is a Y direction. A Z direction in FIGS. 2 and 3 is orthogonal to the X direction and the Y direction.

The nipping part 2 includes a nip roll supporting part 22 and a nip roll motor not illustrated, in addition to the pair of nip rolls 21. The nip roll supporting part 22 rotatably supports each of the pair of nip rolls 21 about the axis 21a. The nip roll motor rotationally drives each of the pair of nip rolls 21 about the axis 21a.

One of the pair of elastic member arrangement parts 3 is an elastic member arrangement part 3 (FIGS. 2, 3, and 5) for the front waistline part F, and the other is an elastic member arrangement part 3 (FIGS. 2 to 4) for the back waistline part B. The elastic member arrangement part 3 for the front waistline part F guides, between the pair of sheets S1 and S2, a plurality of elastic members EL for the front waistline part F (the plurality of upper elastic members EL) among the plurality of elastic members EL. The elastic member arrangement part 3 for the back waistline part B guides, between the pair of sheets S1 and S2, a plurality of elastic members EL for the back waistline part B (the plurality of lower elastic members EL) among the plurality of elastic members EL.

As illustrated in FIGS. 2 and 3, the pair of elastic member arrangement parts 3 is arranged so as to face each other in a direction (Y direction) in which the axes 21a are arranged with reference to a reference plane CP. The reference plane CP is a plane orthogonal to an axis plane AS at the nearest point of the pair of nip rolls 21. The axis plane AS is a plane including each axis 21a of the pair of nip rolls 21. The elastic member arrangement part 3 for the front waistline part F is arranged on the left side in FIG. 2 (right side in FIG. 3), and the elastic member arrangement part 3 for the back waistline part B is arranged on the right side in FIG. 2 (left side in FIG. 3). Since the configuration of each of the pair of elastic member arrangement parts 3 is identical, the elastic member arrangement part 3 for the back waistline part B will be mainly described below.

Each elastic member arrangement part 3 includes a plurality of link members 6a to 6d, a control part 7, and a feeding mechanism 10. The feeding mechanism 10 includes a guiding part 4 and a moving mechanism 5.

The guiding part 4 guides the elastic member EL between the pair of sheets S1 and S2 while feeding the elastic member EL such that the longitudinal direction of the elastic member EL is along the feeding direction. The guiding part 4 is provided upstream of the pair of nip rolls 21 in the conveying direction. The feeding direction is a direction (Z direction) from the guiding part 4 toward the pair of nip rolls 21, and is a direction orthogonal to the axis 21a.

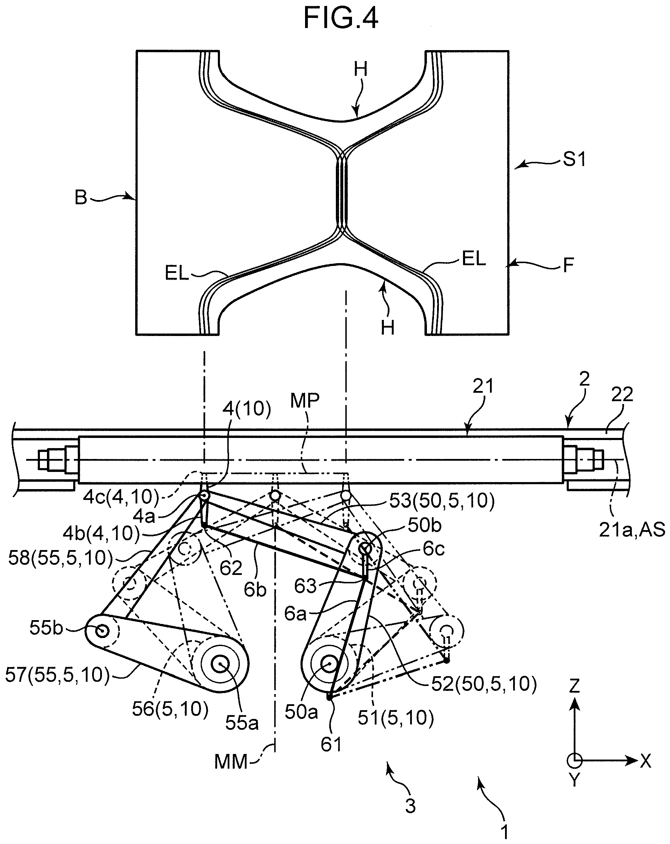

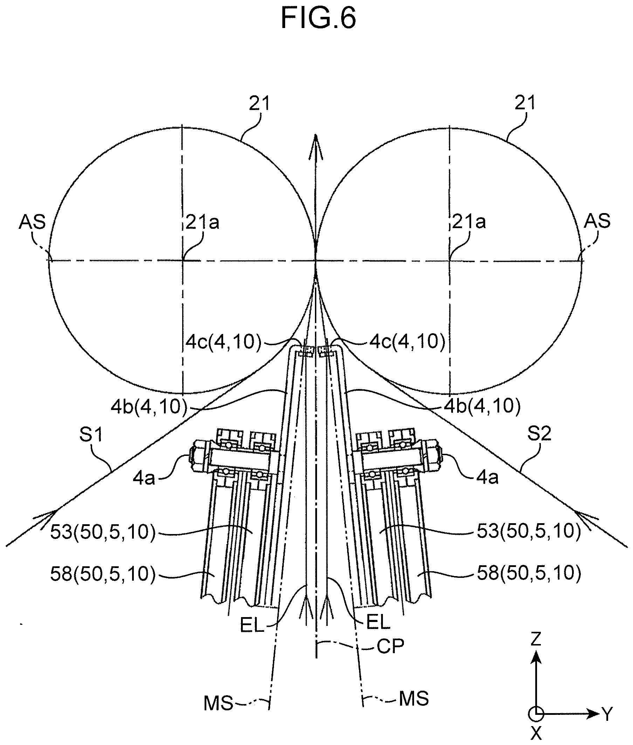

As illustrated in FIGS. 4 and 6, the guiding part 4 has a shape extending in a predetermined direction. The predetermined direction is a direction orthogonal to the axis 21a in the present embodiment. As illustrated in FIG. 6, the guiding part 4 includes a body part 4b extending in the predetermined direction and a holding part 4c. One end part of the body part 4b is provided with the holding part 4c, and the other end part of the body part 4b is connected to a part of the plurality of link members 6a to 6d (a guiding part side link member 6b described later) rotatably about a guiding part link shaft 62 described later. The body part 4b is connected to a part (a first guiding part side arm 53 described later) of the first rotation member 50 rotatably about a guiding part rotation shaft 4a described later between one end part and the other end part of the body part 4b. The holding part 4c holds the elastic member EL so as to be feedable. The holding part 4c extends perpendicularly from one end part of the body part 4b with respect to the end part. The attitude of the guiding part 4 is maintained by a parallel link mechanism (attitude maintaining mechanism) configured to include the plurality of link members 6a to 6d such that the holding part 4c is positioned closer to the nip roll 21 than the moving mechanism 5. This can always maintain the guiding part 4 at a position closer to the nip roll 21 than the moving mechanism 5.

Although not illustrated, a plurality of holding holes penetrating the holding part 4c in a direction (Z direction) orthogonal to the direction in which the pair of axes 21a is arranged is formed in the holding part 4c of the guiding part 4 in order to guide each of the plurality of elastic members EL. The plurality of holding holes are provided side by side in the direction along the axis 21a. In a state where the elastic members EL are inserted into the plurality of respective holding holes, the guiding part 4 guides the plurality of elastic members EL between the pair of sheets S1 and S2. Therefore, the plurality of elastic members EL are guided between the pair of sheets S1 and S2 in a state of being parallel to each other.

The moving mechanism 5 moves the guiding part 4. In the present embodiment, an aspect in which the moving mechanism 5 moves the guiding part 4 includes reciprocation of the guiding part 4 and retraction movement of the guiding part 4 from the pair of nip rolls 21.

As illustrated in FIG. 4, in the reciprocation, the moving mechanism 5 reciprocates the guiding part 4 along a preset moving path MP (FIG. 4). The moving path MP is a reciprocation moving path of the guiding part 4 set such that the plurality of elastic members EL are arranged on the pair of sheets S1 and S2 along a preset arrangement path. The moving path MP extends in a moving direction parallel to a direction (X direction) along the axis 21a. The moving path MP is a straight line in the present embodiment. The moving path MP is included in a moving path plane MS. The moving path plane MS is a plane passing through the holding hole of the holding part 4c and inclined with respect to the reference plane CP in a direction away from the reference plane CP with increasing distance from the pair of nip rolls 21. Furthermore, in the present embodiment, the moving path MP is set such that the distance in a direction orthogonal to the axis 21a between a nip point and the moving path MP becomes constant over the entire moving path MP. The nip point is a position where the plurality of elastic members EL are nipped between the pair of sheets S1 and S2 by the pair of nip rolls 21. The straight line parallel to the axis 21a also includes a curve substantially parallel to the axis 21a.

On the other hand, in the retraction movement, the moving mechanism 5 retracts the guiding part 4 from the pair of nip rolls 21 along a direction orthogonal to the axis 21a.

In order to achieve movement of the guiding part 4, the moving mechanism 5 of the present embodiment includes the first rotation member 50, a first rotation drive part 51, a second rotation member 55, and a second rotation drive part 56.

The first rotation member 50 is rotatable about a first rotation center shaft 50a and is rotatably connected to the guiding part 4. The first rotation member 50 is configured such that a first rotation radius connecting the guiding part 4 and the first rotation center shaft 50a becomes changeable. The first rotation center shaft 50a is orthogonal to the moving path plane MS at a position away from the guiding part 4 in the moving path plane MS.

The first rotation drive part 51 rotates the first rotation member 50 about the first rotation center shaft 50a. Specifically, the first rotation drive part 51 includes a rotation shaft connected to the first rotation member 50, a drive part (not illustrated) that rotationally drives the rotation shaft, a torque detection part 13a (see FIG. 2) that detects a rotation angle of the rotation shaft with respect to the drive part, and an angle detection part 14a (see FIG. 2) that detects torque generated in a rotation shaft. For example, the first rotation drive part 51 includes a servomotor.

The second rotation member 55 is rotatable about a second rotation center shaft 55a, is rotatably connected to the first rotation member 50, and is rotatably connected to the guiding part 4. The second rotation member 55 is configured such that a second rotation radius connecting the guiding part 4 and the second rotation center shaft 55a becomes changeable. The second rotation center shaft 55a extends in parallel with the first rotation center shaft 50a at a position away from the guiding part 4 in the moving path plane MS.

The second rotation drive part 56 rotates the second rotation member 55 about the second rotation center shaft 55a. Specifically, the second rotation drive part 56 includes a rotation shaft connected to the second rotation member 55, a drive part (not illustrated) that rotationally drives the rotation shaft, a torque detection part 13b (see FIG. 2) that detects a rotation angle of the rotation shaft with respect to the drive part, and an angle detection part 14b that detects torque generated in a rotation shaft. For example, the second rotation drive part 56 includes a servomotor.

Rotation of the first rotation member 50 by the first rotation drive part 51 and rotation of the second rotation member 55 by the second rotation drive part 56 are synchronized by a reciprocation control part 7a described later so as to reciprocate the guiding part 4 along the moving path MP. The first rotation member 50 and the second rotation member 55 rotate along the moving path plane MS.

In the present embodiment, the first rotation member 50 and the second rotation member 55 have the identical configuration. The first rotation drive part 51 and the second rotation drive part 56 have the identical configuration. Here, in FIG. 4, the positions of the first rotation member 50 and the second rotation member 55 indicated by broken lines are the positions of the first rotation member 50 and the second rotation member 55 in a state where the guiding part 4 is arranged at a center position of the moving path MP. The center position is a position of a center in a direction along the axis 21a in the moving path MP. Assuming that a straight line passing through the center position and extending in a direction orthogonal to the axis 21a is a center position center line MM, the first rotation center shaft 50a and the second rotation center shaft 55a are arranged at symmetrical positions with reference to the center position center line MM. When the guiding part 4 is positioned at the center position, the first rotation member 50 and the second rotation member 55 are arranged symmetrically with reference to the center position center line MM, and the first rotation drive part 51 and the second rotation drive part 56 are arranged symmetrically.

The first rotation member 50 and the second rotation member 55 will be described more specifically below.

In the present embodiment, the first rotation member 50 includes a first driven arm 52 and the first guiding part side arm 53 that are relatively rotatable with respect to each other. The first driven arm 52 is connected to the first rotation drive part 51 so as to rotate about the first rotation center shaft 50a. The first guiding part side arm 53 is rotatably connected to the first driven arm 52 about a first intermediate shaft 50b. The first intermediate shaft 50b is provided in a part of the first driven arm 52 away from the first rotation center shaft 50a and is parallel to the first rotation center shaft 50a.

The guiding part 4 is rotatably connected to the first guiding part side arm 53 about the guiding part rotation shaft 4a. The guiding part rotation shaft 4a is provided at a part of the first guiding part side arm 53 away from the first intermediate shaft 50b and is parallel to the first rotation center shaft 50a.

In the present embodiment, the first driven arm 52 and the first guiding part side arm 53 have a flat plate shape and are formed of a rigid body.

In the present embodiment, the second rotation member 55 includes a second driven arm 57 and a second guiding part side arm 58 that are relatively rotatable with respect to each other. The second driven arm 57 is connected to the second rotation drive part 56 so as to rotate about the second rotation center shaft 55a. The second guiding part side arm 58 is rotatably connected to the second driven arm 57 about a second intermediate shaft 55b. The second intermediate shaft 55b is provided in a part of the second driven arm 57 away from the second rotation center shaft 55a and is parallel to the first rotation center shaft 50a. Furthermore, the guiding part 4 is rotatably connected to the first guiding part side arm 53 about the guiding part rotation shaft 4a, and is rotatably connected to the second guiding part side arm 58 about the guiding part rotation shaft 4a. The guiding part rotation shaft 4a extends in parallel with the first rotation center shaft 50a in a part of the first guiding part side arm 53 away from the first intermediate shaft 50b, and extends in parallel with the first rotation center shaft 50a in a part of the second guiding part side arm 58 away from the second intermediate shaft 55b. That is, the guiding part rotation shaft 4a is parallel with the first intermediate shaft 50b and the second intermediate shaft 55b.

In the present embodiment, the second driven arm 57 and the second guiding part side arm 58 have a flat plate shape and are formed of a rigid body.

The first guiding part side arm 53 and the second guiding part side arm 58 are rotated by rotation of the first driven arm 52 by the first rotation drive part 51 and rotation of the second driven arm 57 by the second rotation drive part 56. The moving path MP is positioned in a region where the guiding part 4 provided at a tip end parts of the first guiding part side arm 53 and the second guiding part side arm 58 moves.

In the present embodiment, the distance from the first rotation center shaft 50a to the first intermediate shaft 50b and the distance from the second rotation center shaft 55a to the second intermediate shaft 55b are identical, and the distance from the first intermediate shaft 50b to the guiding part rotation shaft 4a and the distance from the second intermediate shaft 55b to the guiding part rotation shaft 4a are identical. That is, the rotation radius of the first driven arm 52 and the rotation radius of the second driven arm 57 are the same, and the rotation radius of the first guiding part side arm 53 and the rotation radius of the second guiding part side arm 58 are the same.

Furthermore, in the present embodiment, at least at the center position, in the direction along the axis 21a, the distance between the guiding part rotation shaft 4a and the first intermediate shaft 50b is longer than the distance between the guiding part rotation shaft 4a and the first rotation center shaft 50a. That is, at least at the center position, the first intermediate shaft 50b is arranged at a position farther from the center position center line MM than the first rotation center shaft 50a in the direction along the axis 21a. Similarly, at least at the center position, in the direction along the axis 21a, the distance between the guiding part rotation shaft 4a and the second intermediate shaft 55b is longer than the distance between the guiding part rotation shaft 4a and the second rotation center shaft 55a. That is, at least at the center position, the second intermediate shaft 55b is arranged at a position farther from the center position center line MM than the second rotation center shaft 55a in the direction along the axis 21a. This can suppress interference between relative rotation of the first guiding part side arm 53 and the first driven arm 52 and relative rotation of the second guiding part side arm 58 and the second driven arm 57.

The plurality of link members 6a to 6d constitute the parallel link mechanism (attitude maintaining mechanism) together with the first driven arm 52 and the first guiding part side arm 53 in order to maintain the attitude of the guiding part 4 such that the holding part 4c is positioned closer to the pair of nip rolls 21 than the moving mechanism 5. The plurality of link members 6a to 6d include a driven link member 6a, the guiding part side link member 6b, an intermediate link member 6c, and a support member 6d.

The support member 6d supports the first rotation center shaft 50a and the link rotation center shaft 61 so as to restrict relative movement with the first rotation center shaft 50a and the link rotation center shaft 61. The link rotation center shaft 61 is parallel to the first rotation center shaft 50a. In the present embodiment, the link rotation center shaft 61 is supported by the support member 6d at a position away from the first rotation center shaft 50a. The direction in which the link rotation center shaft 61 is away from the first rotation center shaft 50a is a direction orthogonal to the axis 21a and away from the pair of nip rolls 21.

The driven link member 6a is supported by the support member 6d in a state of being rotatable about the link rotation center shaft 61.

The guiding part side link member 6b is connected to the guiding part 4 rotatably about the guiding part link shaft 62, and is connected rotatably about a first intermediate link shaft 63 with respect to the driven link member 6a. The guiding part link shaft 62 is parallel to the first rotation center shaft 50a. The first intermediate link shaft 63 is parallel to the first rotation center shaft 50a.

The intermediate link member 6c is supported by the first guiding part side arm 53 rotatably about the first intermediate shaft 50b, and is connected rotatably about the first intermediate link shaft 63 with respect to the driven link member 6a and the guiding part side link member 6b.

By the plurality of link members 6a to 6d, the first driven arm 52, and the first guiding part side arm 53, the parallel link mechanism constitutes two parallelograms in the present embodiment.

One parallelogram of the two parallelograms is formed by a straight line connecting the guiding part rotation shaft 4a and the first intermediate shaft 50b, a straight line connecting the guiding part link shaft 62 and the first intermediate link shaft 63, a straight line connecting the guiding part rotation shaft 4a and the guiding part link shaft 62, and a straight line connecting the first intermediate shaft 50b and the first intermediate link shaft 63. The other parallelogram of the two parallelograms is formed by a straight line connecting the first rotation center shaft 50a and the first intermediate shaft 50b, a straight line connecting the link rotation center shaft 61 and the first intermediate link shaft 63, a straight line connecting the first rotation center shaft 50a and the link rotation center shaft 61, and a straight line connecting the first intermediate shaft 50b and the first intermediate link shaft 63.

As illustrated in FIG. 2, the plurality of link members 6a to 6d face each other across the reference plane CP in each of the pair of elastic member arrangement parts 3. Therefore, the pair of elastic member arrangement parts 3 is inclined in a direction away from each other with increasing distance from the pair of nip rolls 21 so that the plurality of link members 6a to 6d of each elastic member arrangement part 3 do not come into contact with each other.

The control part 7 controls movement of the moving mechanism 5 including the reciprocation and the retraction movement. The control part 7 includes the reciprocation control part 7a and a retraction movement control part 7b.

The reciprocation control part 7a performs reciprocation control for synchronizing rotation of the first rotation member 50 by the first rotation drive part 51 and rotation of the second rotation member 55 by the second rotation drive part 56 so as to reciprocate the guiding part 4 along the moving path MP. That the reciprocation control part 7a synchronizes rotation of the first rotation member 50 with rotation of the second rotation member 55 means that the reciprocation control part 7a determines a first rotation angle of the first rotation member 50 and a second rotation angle of the second rotation member 55 so as to move the guiding part 4 along the moving path MP, and the reciprocation control part 7a controls rotation of the first rotation drive part 51 and the second rotation drive part 56 based on the first rotation angle and the second rotation angle. The first rotation angle is a rotation angle about the first rotation center shaft 50a of the straight line connecting the first rotation center shaft 50a and the first intermediate shaft 50b. The second rotation angle is a rotation angle about the second rotation center shaft 55a of a straight line connecting the second rotation center shaft 55a and the second intermediate shaft 55b.

Reciprocation of the guiding part 4 along the moving path MP is achieved by the reciprocation control. The reciprocation control will be specifically described as follows. First, in FIG. 4, the positions of the first rotation member 50 and the first rotation drive part 51, and the second rotation member 55 and the second rotation drive part 56 indicated by solid lines are assumed to be origin positions. The origin position is a position of the moving mechanism 5 and the guiding part 4 in a state where the guiding part 4 is positioned at one end part of the moving path MP in the direction (X direction) along the axis 21a. In the present embodiment, the guiding part 4 is positioned at the leftmost point of the moving path MP at the origin position. In FIG. 4, a position where the first rotation member 50 and the first rotation drive part 51 and the second rotation member 55 and the second rotation drive part 56 indicated by two-dot chain lines are arranged symmetrically with the origin position with respect to the center position center line MM is assumed to be a turn-back position. The turn-back position is a position of the moving mechanism 5 and the guiding part 4 in a state where the guiding part 4 is positioned at the other end part of the moving path MP in the direction along the axis 21a. In the present embodiment, the guiding part 4 is positioned at the rightmost point of the moving path MP at the turn-back position.

In order to move the guiding part 4 from the origin position of the moving path MP to the turn-back position via the center position, the reciprocation control part 7a makes the rotation direction of the first rotation member 50 and the rotation direction of the second rotation member 55 identical to each other. Specifically, the reciprocation control part 7a determines the first rotation angle in a clockwise direction of the first driven arm 52 and the second rotation angle in a clockwise direction of the second driven arm 57 so as to change in time series. The first rotation angle and the second rotation angle are determined such that the guiding part 4 can move along the moving path MP.

Then, the reciprocation control part 7a rotates the first rotation drive part 51 along time series in the clockwise direction at the first rotation angle, and rotates the second rotation drive part 56 along time series in the clockwise direction at the second rotation angle.

Due to this, the first driven arm 52 and the second driven arm 57 rotate in synchronization with each other in a state where both the first guiding part side arm 53 and the second guiding part side arm 58 are connected to the guiding part 4, whereby the guiding part 4 can be reciprocated along the moving path MP with relative rotation of the first guiding part side arm 53 with respect to the first driven arm 52 and relative rotation of the second guiding part side arm 58 with respect to the second driven arm 57. In relative rotation of the first guiding part side arm 53 with respect to the first driven arm 52, an angle formed by the first driven arm 52 and the first guiding part side arm 53 changes, whereby a first distance between the first rotation center shaft 50a and the guiding part rotation shaft 4a changes in the first rotation radius direction. A second distance between the second rotation center shaft 55a and the guiding part rotation shaft 4a changes in the second rotation radius direction by relative rotation of the second guiding part side arm 58 with respect to the second driven arm 57. When the guiding part 4 is moved from the origin position to the turn-back position via the center position, the first distance changes so as to become short in the first rotation radius direction and the second distance changes so as to become long in the second rotation radius direction.

Furthermore, in order to move the guiding part 4 from the turn-back position to the origin position via the center position of the moving path MP, the reciprocation control part 7a determines the first rotation angle in a counterclockwise direction of the first driven arm 52 and the second rotation angle in a counterclockwise direction of the second driven arm 57 so as to change in time series.

Then, the reciprocation control part 7a rotates the first rotation drive part 51 along time series in the counterclockwise direction at the first rotation angle, and rotates the second rotation drive part 56 along time series in the counterclockwise direction at the second rotation angle.

Due to this, in a state where both the first guiding part side arm 53 and the second guiding part side arm 58 are connected to the guiding part 4, the first distance changes so as to become long in the first rotation radius direction and the second distance changes so as to become short in the second rotation radius direction. Accordingly, the guiding part 4 is moved along the origin position from the turn-back position of the moving path MP via the center position.

By the reciprocation of the moving path MP of the guiding part 4 described above, the back waistline part B in a state where the elastic member EL is nipped between the pair of sheets S1 and S2 is manufactured as illustrated in FIG. 4.

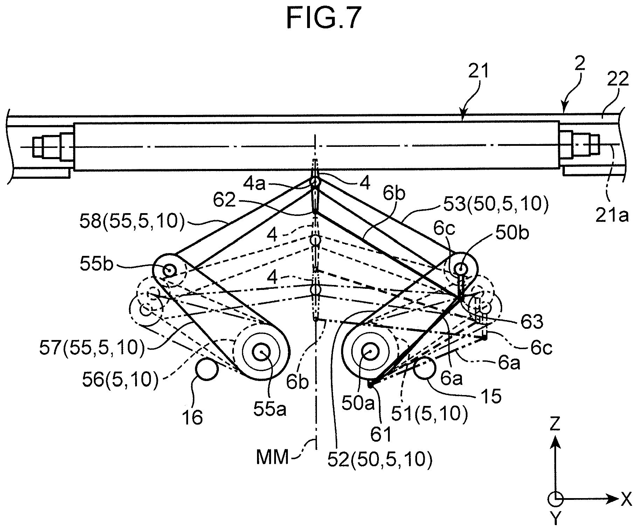

As illustrated in FIG. 7, the retraction movement control part 7b performs retraction movement control of synchronizing rotation of the first rotation member 50 and rotation of the second rotation member 55 so as to retract the guiding part 4 from the pair of nip rolls 21 along the direction orthogonal to the axis 21a. The retraction movement control is control performed in a period other than a period in which the reciprocation control is performed. That the retraction movement control part 7b synchronizes rotation of the first rotation member 50 with rotation of the second rotation member 55 means that the retraction movement control part 7b determines the first rotation angle and the second rotation angle so as to retract the guiding part 4 from the pair of nip rolls 21, and the retraction movement control part 7b controls rotation of the first rotation drive part 51 and second rotation drive part 56 based on the first rotation angle and the second rotation angle.

In the present embodiment, the retraction movement control is executed when the guiding part 4 exists at the center position as illustrated in FIG. 7. The retraction movement control will be specifically described as follows. In order to move the guiding part 4 from a position close to the pair of nip rolls 21 to a position away from the pair of nip rolls 21 in the retraction movement control, the retraction movement control part 7b makes a rotation direction of the first rotation member 50 and a rotation direction of the second rotation member 55 different. Specifically, the retraction movement control part 7b determines the first rotation angle in the clockwise direction of the first driven arm 52 and the second rotation angle in the counterclockwise direction of the second driven arm 57 so as to change in time series. In the present embodiment, the first rotation angle and the second rotation angle in the retraction movement control are determined to be identical.

Then, the retraction movement control part 7b rotates the first rotation drive part 51 along time series in the clockwise direction at the first rotation angle, and rotates the second rotation drive part 56 along time series in the counterclockwise direction at the second rotation angle.

Due to this, the first driven arm 52 and the second driven arm 57 rotate in synchronization with each other in a state where both the first guiding part side arm 53 and the second guiding part side arm 58 are connected to the guiding part 4. Therefore, the guiding part 4 is moved away from the pair of nip rolls 21 with relative rotation of the first guiding part side arm 53 with respect to the first driven arm 52 and relative rotation of the second guiding part side arm 58 with respect to the second driven arm 57. Note that as illustrated in FIG. 7, the manufacturing device 1 includes a stopper 15 that restricts clockwise rotation of the first driven arm 52 and a stopper 16 that restricts counterclockwise rotation of the second driven arm 57. In order to retract the guiding part 4 from the nip roll 21, the retraction movement control part 7b rotates both the driven arms 52 and 57 to a position (hereinafter, referred to as a retraction position) where both the driven arms 52 and 57 come into contact with both the stoppers 15 and 16.

An interval between the guiding part 4 and the pair of nip rolls 21 can be increased by retracting the guiding part 4 from the pair of nip rolls 21. Maintenance of the feeding mechanism 10 can be performed using the interval.

Hereinafter, processing executed by the control part 7 at the time of manufacturing the elastic laminate L will be described with reference to FIGS. 2 and 8 to 11.

As illustrated in FIG. 2, the manufacturing device 1 for the elastic laminate L further includes a storage part 11 and an input operation part 12 electrically connected to the control part 7.

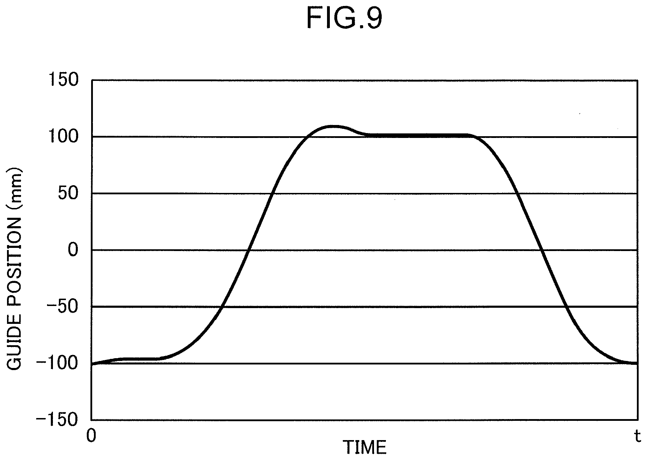

The storage part 11 stores a movement pattern (FIG. 9) of the guiding part 4 of the moving path MP different according to a specification of the elastic laminate L (the size of the disposable diaper D in the present embodiment) for each specification. Furthermore, the storage part 11 stores a drive pattern of the first rotation drive part 51 set based on the movement pattern of FIG. 9 and a drive pattern of the second rotation drive part 56 set based on the movement pattern of FIG. 9. FIGS. 9 to 10 include a pattern of one cycle for reciprocating on the moving path MP.

The input operation part 12 allows an operation for inputting a command for selecting one specification of a plurality of specifications of the elastic laminate L, and an operation for inputting a command for starting manufacturing and a command for stopping manufacturing of the elastic laminate L. For example, the input operation part 12 includes a touchscreen.

As illustrated in FIG. 2, the control part 7 is electrically connected also to the torque detection parts 13a and 13b and the angle detection parts 14a and 14b in the first rotation drive part 51 and the second rotation drive part 56 described above. The control part 7 executes processing for manufacturing the elastic laminate L based on information from the storage part 11, the input operation part 12, the torque detection parts 13a and 13b, and the angle detection parts 14a and 14b.

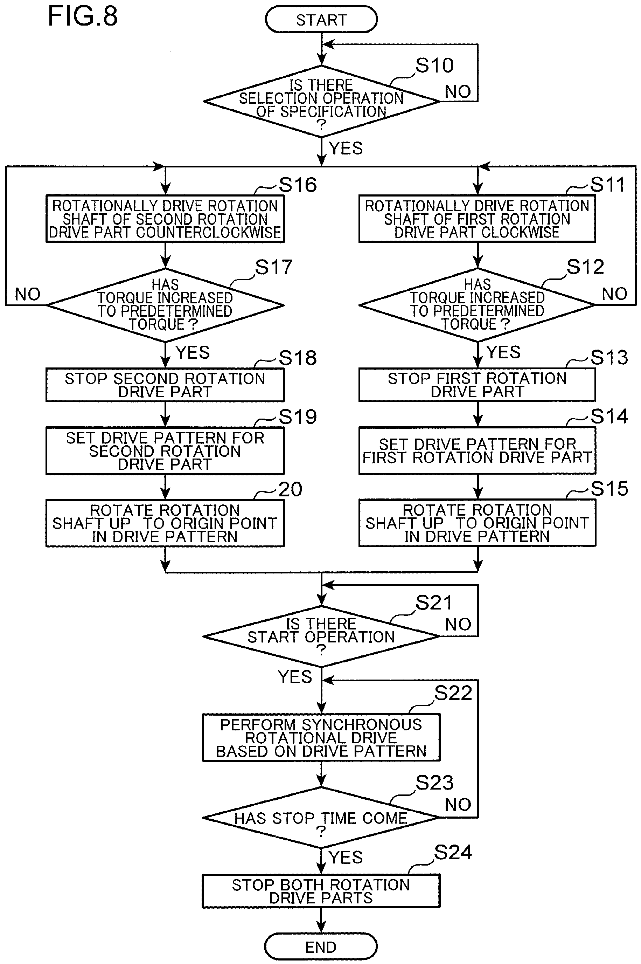

Specifically, as illustrated in FIG. 8, when processing by the control part 7 is started, the process waits for a selection operation of the specification of the elastic laminate L (the specification of the disposable diaper D in the present embodiment) (step S10).

When it is determined in step S10 that the selection operation of the specification of the elastic laminate L has been performed (YES in step S10), processing for aligning the rotation shaft of the first rotation drive part 51 with the origin (steps S11 to S15) and processing for aligning the rotation shaft of the second rotation drive part 56 with the origin (steps S16 to S20) are executed.

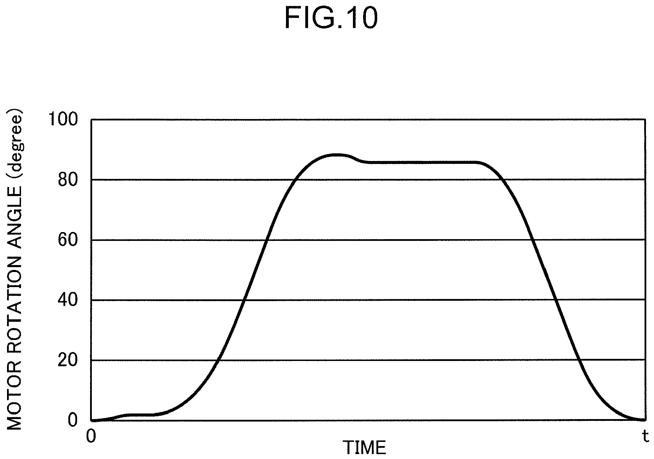

As illustrated in FIGS. 7 and 8, the first rotation drive part 51 rotationally drives the rotation shaft clockwise (step S11), and determines whether the torque has increased to predetermined torque by the first driven arm 52 coming into contact with the stopper 15 (step S12). When it is determined that the torque is less than the predetermined torque based on the detection result of the torque detection part 13a (NO in step S12), step S11 is continuously executed. On the other hand, when it is determined that the torque has increased to the predetermined torque (YES in step S12), the first rotation drive part 51 is stopped (step S13). Note that the rotation position of the first rotation drive part 51 stopped in this manner is stored as a reference position in the storage part 11, and the first rotation drive part 51 is controlled so as to have a "motor rotation angle" in a drive pattern (e.g., that described in FIG. 10) based on a relative rotation angle (detection angle by the angle detection part 14a) from this reference position. Specifically, the pattern of the first rotation drive part 51 is read from the storage part 11 based on the specification selected in step S10, this is set as a current driving pattern (step S14), and the first rotation drive part 51 is rotationally driven to the origin position (e.g., the position where the "motor rotation angle" is 0 in FIG. 10) set in the pattern (step S15).

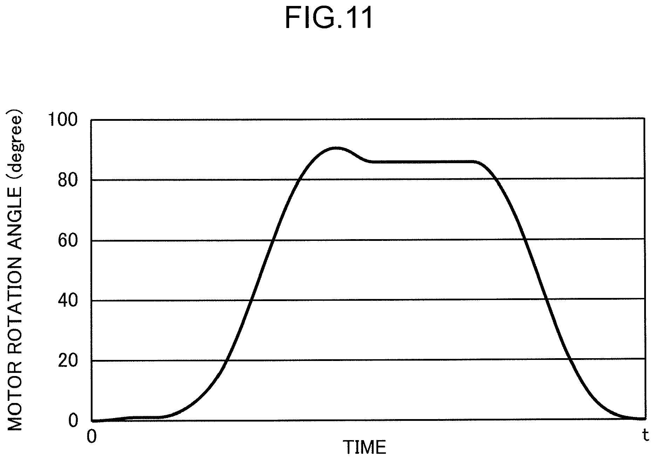

On the other hand, the second rotation drive part 56 rotationally drives the rotation shaft counterclockwise (step S16), and determines whether the torque has increased to predetermined torque by the second driven arm 57 coming into contact with the stopper 16 (step S17). When it is determined that the torque is less than the predetermined torque based on the detection result by the torque detection part 13b (NO in step S17), step S16 is continuously executed. On the other hand, when it is determined that the torque has increased to the predetermined torque (YES in step S17), the second rotation drive part 56 is stopped (step S18). Similarly to the first rotation drive part 51, the rotation position of the second rotation drive part 56 stopped in this manner is stored as a reference position in the storage part 11, and the second rotation drive part 56 is controlled to have a "motor rotation angle" in a drive pattern (e.g., that described in FIG. 11) based on a relative rotation angle (detection angle by the angle detection part 14b) from this reference position. Specifically, the pattern of the second rotation drive part 56 is read from the storage part 11 based on the specification selected in step S10, this is set as a current driving pattern (step S19), and the second rotation drive part 56 is rotationally driven to the origin position (e.g., the position where the "motor rotation angle" is 0 in FIG. 11) set in the pattern (step S20).

When the processing (steps S11 to S20) of aligning the origin with respect to the rotation shafts of the rotation drive parts 51 and 56 is completed in this manner, the process waits for a start operation of manufacturing of the elastic laminate L to be performed by the input operation part 12 (see FIG. 2) (step S21).

When it is determined that there is an operation for starting manufacturing (YES in step S21), both the rotation drive parts 51 and 56 are synchronously rotationally driven based on the drive patterns set in steps S14 and S19 (step S22). Specifically, control part 7 controls drive of both the rotation drive parts 51 and 56 along a common time axis using two drive patterns (e.g., those illustrated in FIGS. 10 and 11) for both the rotation drive parts 51 and 56. Note that in FIGS. 10 and 11, a drive pattern for one cycle for reciprocating once on the moving path is set, and the control part 7 repeatedly executes the same drive pattern from the beginning when one cycle ends for a specific drive pattern.

Next, it is determined whether a preset stop time has come (step S23). The stop time may be automatically generated in response to input of the start operation described above, or may be manually input with the input operation part 12. Until the stop time comes (while NO in step S23), the synchronous rotational drive is continued (step S22).

On the other hand, when it is determined that the stop time has come (YES in step S23), both the rotation drive parts 51 and 56 are stopped (step S24), and the processing is ended.

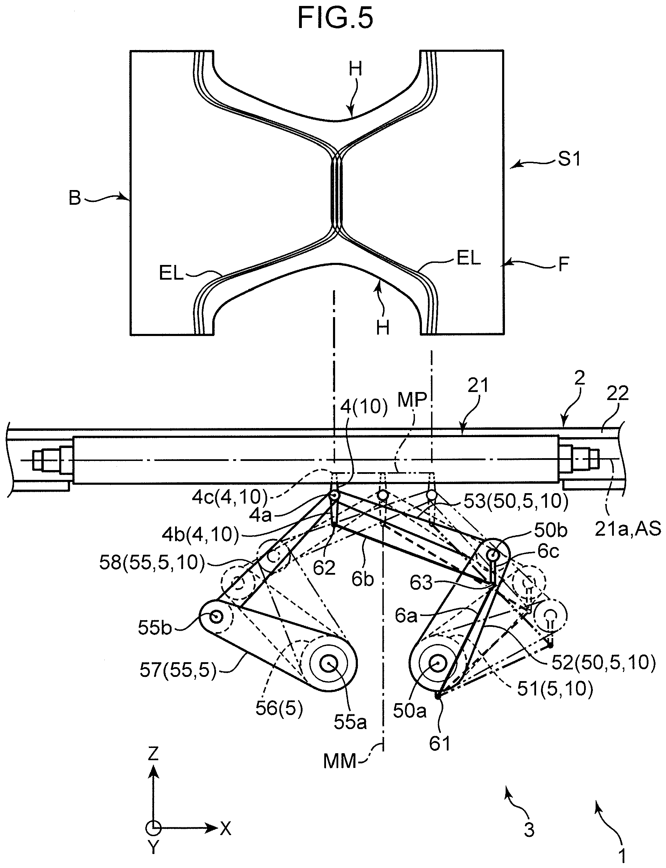

Of the pair of elastic member arrangement parts 3, the elastic member arrangement part 3 for the front waistline part F illustrated in FIG. 5 has an identical configuration to the elastic member arrangement part 3 for the back waistline part B illustrated in FIGS. 2, 3, and 4 described above. The elastic member arrangement part 3 for the front waistline part F arranges the elastic member EL for the front waistline part F in the front waistline part F by a procedure similar to that of the elastic member arrangement part 3 for the back waistline part B.

On the other hand, the distance of the moving path for moving the guiding part 4 in the elastic member arrangement part 3 for the front waistline part F is different from the distance of the moving path MP for moving the guiding part 4 in the elastic member arrangement part 3 for the back waistline part B. In the present embodiment, the elastic member arrangement part 3 for the front waistline part F has a length of the moving path of the guiding part 4 set to be shorter than a length of the moving path MP of the elastic member arrangement part 3 for the back waistline part B. Accordingly, the reciprocation control part 7a sets a maximum angle of the first rotation angle and a maximum angle of the second rotation angle to be smaller than those in a case of manufacturing the back waistline part B.

Next, a manufacturing method for the elastic laminate L will be described. The manufacturing method for the elastic laminate L includes (1) a manufacturing device preparation process, (2) a sheet conveyance process, (3) an elastic member arrangement process, and (4) a retraction process.

### (1) Manufacturing Device Preparation Process

In the manufacturing device preparation process, the manufacturing device 1 is prepared.

The pair of sheets S1 and S2 is prepared, and the end parts in the sheet longitudinal direction of the pair of sheets S1 and S2 are nipped between the pair of nip rolls 21. Specifically, as illustrated in FIG. 2, the sheet S1 is nipped between the pair of nip rolls 21 from one side of the pair of nip rolls 21, and the sheet S2 is nipped between the pair of nip rolls 21 from the other side of the pair of nip rolls 21.

A pair of the plurality of elastic members EL is guided through between the pair of elastic member arrangement parts 3. Each of the pair of the plurality of elastic members EL is guided to the guiding part 4 of the pair of elastic member arrangement parts 3, respectively. The plurality of elastic members EL are inserted into the plurality of holding holes, respectively, of the holding part 4c in the guiding part 4.

### (2) Sheet Conveyance Process

In the sheet conveyance process, a drive roller not illustrated provided in a conveyance path of the sheets S1 and S2 is rotationally driven, and the pair of nip rolls 21 is rotationally driven, thereby conveying the pair of sheets S1 and S2 in the conveying direction.

### (3) Elastic Member Arrangement Process

In the elastic member arrangement process, the plurality of elastic members EL are guided between the pair of sheets S1 and S2, and the elastic laminate L in which the plurality of elastic members EL are nipped between the pair of sheets S1 and S2 is manufactured.

Specifically, the reciprocation control part 7a synchronizes rotation of the first rotation member 50 by the first rotation drive part 51 and rotation of the second rotation member 55 by the second rotation drive part 56 so as to reciprocate the guiding part 4 along the moving path MP. In the present embodiment, the first driven arm 52 and the second driven arm 57 are rotated together with rotation of the first guiding part side arm 53 and the second guiding part side arm 58, thereby moving the guiding part 4 along the moving path MP.

The plurality of elastic members EL are fed from the guiding part 4 in the longitudinal direction of the plurality of elastic members EL between the pair of sheets S1 and S2 in a state where the guiding part 4 is reciprocated along the moving path MP.

Due to this, the plurality of elastic members EL are conveyed while being nipped between the pair of nip rolls 21 in a state of being interposed between the pair of sheets S1 and S2, and the elastic laminate L is manufactured.

In the present embodiment, the plurality of link members 6a to 6d enable the guiding part 4 to move along the moving path MP in a state of maintaining the attitude. A specific explanation is as follows. In the parallel link mechanism, the first rotation center shaft 50a and the link rotation center shaft 61 are supported by the support member 6d in a state where relative movement is restricted. Due to this, the attitude of the guiding part 4 during the movement period of the guiding part 4 by the moving mechanism 5, that is, the orientation of the line connecting the guiding part rotation shaft 4a and the guiding part link shaft 62 is maintained in a state of matching the orientation of a line connecting the first rotation center shaft 50a and the link rotation center shaft 61. That is, the guiding part 4 can be moved along the moving path MP while maintaining the attitude corresponding to the orientations of these straight lines.

In the present embodiment, the moving path MP is set such that the distance in the feeding direction (direction orthogonal to the axis 21a) between the nip point and the moving path MP becomes constant over the entire moving path MP. When the moving path MP of the guiding part 4 with respect to an arrangement path of the elastic member EL is set, the moving path MP needs to be set so as to move the guiding part 4 excessively according to the distance from the guiding part 4 to the nip point. That is, a time difference corresponding to the distance between the guiding part 4 and the nip point is generated between when a predetermined part of the elastic member EL is fed from the guiding part 4 and when the predetermined part actually reaches the nip point. Therefore, in order to arrange the elastic member EL at a desired nip point, it is necessary to move the guiding part 4 in the moving direction in advance in consideration of the time difference. Here, when the distance between the nip point and the moving path MP changes, the time difference varies depending on the position of the guiding part 4, and thus complicated calculation is required for setting or changing the moving path MP. According to the above, since the distance in the feeding direction between the nip point and the moving path MP is constant over the entire moving path MP, the time difference becomes constant, and calculation for setting the moving path MP is easy.

In the present embodiment, since the rotation radius of the first driven arm 52 and the rotation radius of the second driven arm 57 are the same, and the rotation radius of the first guiding part side arm 53 and the rotation radius of the second guiding part side arm 58 are the same, when the moving path MP is set such that the center position of the moving path MP is positioned on a straight line passing through the center position in a direction along the axis 21a between the first rotation center shaft 50a and the second rotation center shaft 55a, operations of the first rotation drive part 51 and the second rotation drive part 56 can be set symmetrically with reference to the center position, and hence it is possible to simplify calculation processing when rotation of the first rotation drive part 51 and rotation of the second rotation drive part 56 are synchronized with each other.

In the elastic member arrangement process, an adhesive may be applied to at least any of the pair of sheets S1 and S2 and the plurality of elastic members EL. The pair of sheets S1 and S2 and the plurality of elastic members EL may be welded by applying heat to the elastic laminate L in a state where the plurality of elastic members EL are interposed between the pair of sheets S1 and S2. The heat may be applied, for example, by the pair of nip rolls 21 heated or by ultrasonically vibrating the pair of nip rolls.

### (4) Retraction Process

In the retraction process, first, the guiding part 4 is moved to the center position. Next, the retraction movement control part 7b synchronizes rotation of the first rotation member 50 and rotation of the second rotation member 55 so as to retract the guiding part 4 from the pair of nip rolls 21 along the direction orthogonal to the axis 21a.

According to the manufacturing device 1 and the manufacturing method for the elastic laminate L, the guiding part 4 can be reciprocated along the moving path MP without using a restriction mechanism accompanied by sliding friction of the slider with respect to the rail. This can reduce maintenance frequency. Specifically, in a state where the first rotation member 50 and the second rotation member 55 are rotatably connected to each other, the moving mechanism 5 is controlled so that rotation by the first rotation drive part 51 and rotation by the second rotation drive part 56 are synchronized so as to move the guiding part 4 along the moving path MP, whereby the guiding part 4 can be reciprocated along the moving path MP with a change in the first rotation radius of the first rotation member 50 and a change in the second rotation radius of the second rotation member 55. This can omit the restriction mechanism, and can reduce the frequency of maintenance due to existence of the restriction mechanism.

Since sliding resistance of the moving mechanism at the time of reciprocating the guiding part 4 is small as compared with a case where the guiding part 4 reciprocates by the belt bridged on the pair of pulleys reciprocating so as to repeat reversal of the moving direction as conventionally, the moving speed of the guiding part 4 can be increased, and the manufacturing speed of the elastic laminate L can be improved.

Note that the present invention is not limited to the above embodiment, and for example, the following aspects can also be adopted.

### (A) First Rotation Member and Second Rotation Member

The first rotation member and the second rotation member of the present invention are each not limited to an aspect configured by connection of a plurality of arms as long as it is an aspect in which the first distance and the second distance change according to rotation about the first rotation center shaft and the second rotation center shaft. As an aspect in which the first distance and the second distance change according to rotation, the first rotation member and the second rotation member of the present invention may include an expandable member, for example, a telescopic member. A telescopic member includes two members including, for example, a housing member and a housed member. The telescopic member is expandable by being configured to be capable of pulling out or housing the housed member with respect to the housing member.

Specifically, the first rotation member includes a first housing member and a first housed member connected to the first housing member so as to be capable of being pulled out or housed. The second rotation member includes a second housing member and a second housed member connected to the second housing member so as to be capable of being pulled out or housed. The first housing member is connected to the first rotation center shaft. The first housed member is connected to the guiding part. The second housing member is connected to the second rotation center shaft. The second housed member is connected to the guiding part. When the first housing member rotates about the first rotation center shaft and the second housing member rotates about the second rotation center shaft in a state where both the first housed member and the second housed member are connected to the guiding part, the first housed member is pulled out or housed with respect to the first housing member, and the second housed member is pulled out or housed with respect to the second housing member. Due to this, the first distance and the second distance change so as to move the guiding part along the moving path.

### (B) Relationship between First Rotation Center Shaft and Second Rotation Center Shaft and Direction Orthogonal to Axis 21a

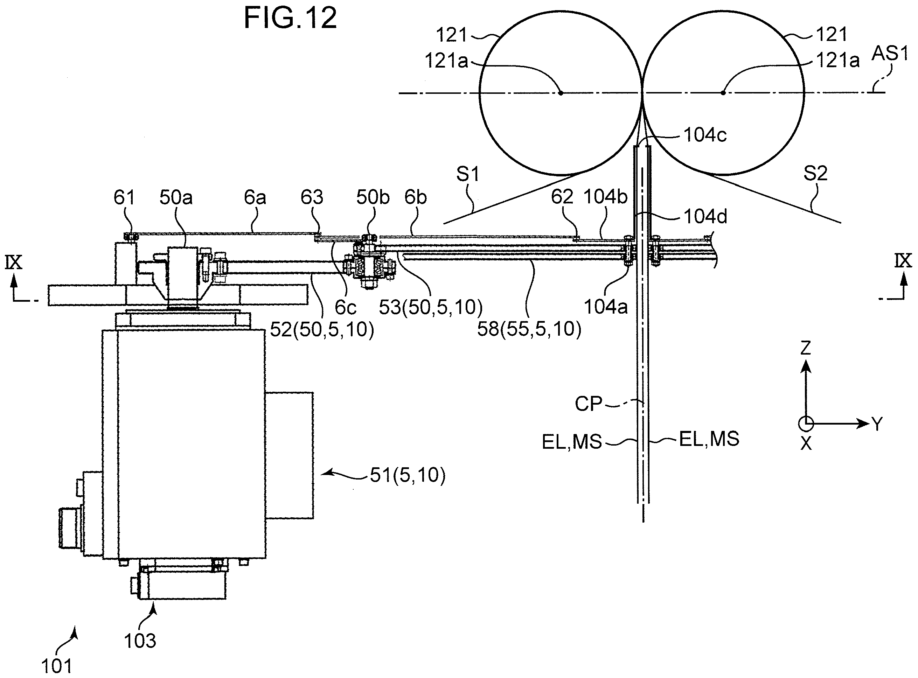

In the above embodiment, the first rotation center shaft and the second rotation center shaft of the present invention extend along the direction (Y direction) in which the pair of axes 21a is arranged. That is, the first rotation center shaft 50a and the second rotation center shaft 55a of the above embodiment are parallel to the axis plane AS as illustrated in FIG. 2. However, the first rotation center shaft and the second rotation center shaft of the present invention may extend in a direction (Z direction) orthogonal to the direction in which the pair of axes 21a is arranged. This will be described below with reference to FIG. 12.

A manufacturing device 101 illustrated in FIG. 12 includes a pair of nip rolls 121 including an axis 121a and a pair of elastic member arrangement parts 103. Each elastic member arrangement part 103 includes the plurality of link members 6a to 6d, the control part 7, and the feeding mechanism 10. The feeding mechanism 10 includes a guiding part 104 and the moving mechanism 5. The guiding part 104 is different in configuration from the guiding part 4 of the above embodiment, but the moving mechanism 5, the plurality of link members 6a to 6d, and the control part 7 have the same configurations as those of the above embodiment. The elastic member arrangement part 103 is arranged with respect to the pair of nip rolls 121 such that the first rotation center shaft 50a of the first rotation member 50 of the moving mechanism 5 extends in a direction orthogonal to the direction in which the pair of axes 21a is arranged.

The guiding part 104 guides the plurality of elastic members EL toward the pair of nip rolls 121. Specifically, the guiding part 104 includes a first body part 104b extending along an axis plane AS 1, a second body part 104d extending in a direction orthogonal to the axis plane AS 1 from the first body part 104b toward the pair of nip rolls 121, and a holding part 104c provided at an end part close to the pair of nip rolls 121 of the second body part 104d. The holding part 104c extends from the second body part 104d in a direction orthogonal to the second body part 104d. The axis plane AS1 is a plane including a pair of the axes 121a and is along a direction orthogonal to the axis 21a.

In the manufacturing device 101, the first rotation center shaft 50a in FIG. 12 extends in a direction orthogonal to the direction in which the pair of axes 21a is arranged, that is, orthogonal to the axis plane AS 1. Although not illustrated, the second rotation center shaft is also orthogonal to the axis plane AS1. In the manufacturing device 101, the first rotation member 50 and the second rotation member rotate along the axis plane AS 1. Furthermore, the holding part 104c also rotates along the axis plane AS1. Therefore, the moving path of the holding part 104c is along the axis plane AS 1. The plurality of elastic members are guided between the pair of sheets by the guiding part 104 moving along the moving path.

### (C) Moving Path

### (C1) Shape of Trajectory of Moving Path

The moving path is not limited to a straight line parallel to the axis, and may be a curve. For example, a trajectory of the nip point may be curved over the axis direction, and the moving path may be curved over the axis direction similarly to the trajectory of the nip point.

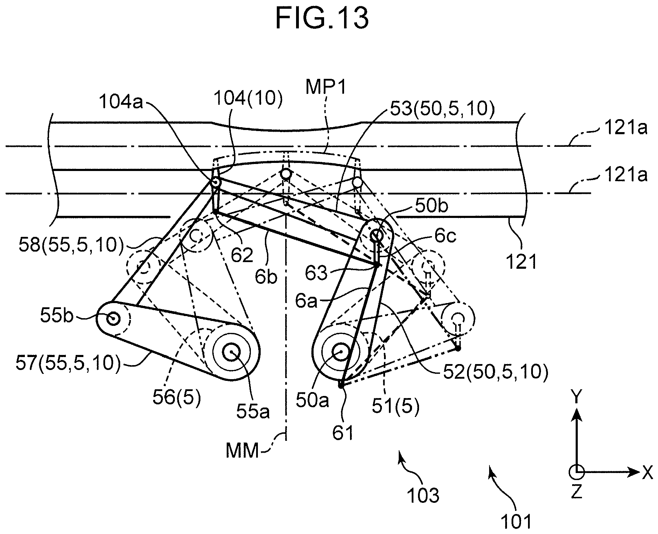

The aspect in which the moving path and the trajectory of the nip point are curved can be achieved by curving outer surfaces of the pair of nip rolls 121 in the manufacturing device 101 illustrated in FIG. 12. This will be described below with reference to FIG. 13 together with FIG. 12.

The outer surfaces of the pair of nip rolls 121 of the manufacturing device 101 illustrated in FIG. 12 can have a curved form as illustrated in FIG. 13. Specifically, as illustrated in FIG. 13, the outer surface of one nip roll 121 (lower nip roll 121 in FIG. 13) of the pair of nip rolls 121 is curved in a convex shape along the axis 121a of the one nip roll 121. The outer surface of the other nip roll 121 (upper nip roll 121 in FIG. 13) of the pair of nip rolls 121 is curved in a concave shape along the axis 121a of the other nip roll 121 so as to mesh with the outer surface of the one nip roll 121. The pair of nip rolls 121 can nip each other the pair of sheets S1 and S2 and the plurality of elastic members EL by the outer surface in the convex shape of the one nip roll 121 and the outer surface in the concave shape of the other nip roll 121.

The guiding part 104 feeds the plurality of elastic members EL while moving along a curved moving path MP1. The curved moving path MP1 is a moving path of the guiding part 104. The curved moving path MP1 is set so as to be curved over a direction in which the axis 121a extends in accordance with the curved shape of the pair of nip rolls 121. In the example of FIG. 13, the curved moving path MP1 is curved in an arc shape.

The plurality of elastic members EL fed from the guiding part 104 are guided between the pair of sheets S1 and S2. The pair of sheets S1 and S2 and the plurality of elastic members EL are conveyed while being nipped between the pair of nip rolls 121 in a state of being curved in the direction in which the axis 121a extends. Due to this, the trajectory of the nip point of the plurality of elastic members EL is curved over the direction in which the axis 121a extends in accordance with the curved shape of the pair of nip rolls 121. It is preferable that the distance in a direction orthogonal to the axis 121a between the curved moving path MP1 and the nip point is constant over the direction in which the axis 121a extends.

The reciprocation control part 7a controls the moving mechanism 5 such that the guiding part 104 reciprocates along the curved moving path MP1.

Note that the moving path is not limited to a linear shape and an arc shape, and can be a route having various shapes. That is, the moving path can be arbitrarily set within a region where the guiding part positioned at the tip ends of the first guiding side arm and the second guiding side arm can move by synchronization by the reciprocation control part of rotation of the first rotation member by the first rotation drive part and rotation of the second rotation member by the second rotation drive part.

### (C2) Distance between Nip Point and Moving Path

The moving path is not limited to an aspect in which the distance in a direction orthogonal to the axis between the nip point and the moving path is necessarily constant over the direction in which the axis extends, and the distance may change over the direction in which the axis extends. For example, the trajectory of the nip point may be curved over the direction in which the axis extends, and the moving path may be a straight line over the direction in which the axis extends. Conversely, the trajectory of the nip point may be a straight line over the direction in which the axis extends, and the moving path may be curved over the direction in which the axis extends.

### (D) Attitude Maintaining Mechanism

### (D1) Another Example of Attitude Maintaining Mechanism

The attitude maintaining mechanism of the present invention is not limited to the parallel link mechanism, and for example may be a correction mechanism that corrects the attitude of the guiding part, for example. The correction mechanism detects a shift in the predetermined direction in which the guiding part 4 extends with respect to the direction orthogonal to the axis, for example, and corrects the shift, thereby rotating the guiding part so as to maintain the attitude of the guiding part.

The plurality of link members of the present invention are not limited to an aspect provided in the first rotation member and the first rotation drive part, and may be further provided in the second rotation member and the second rotation drive part.

### (D2) Omission of Link Member

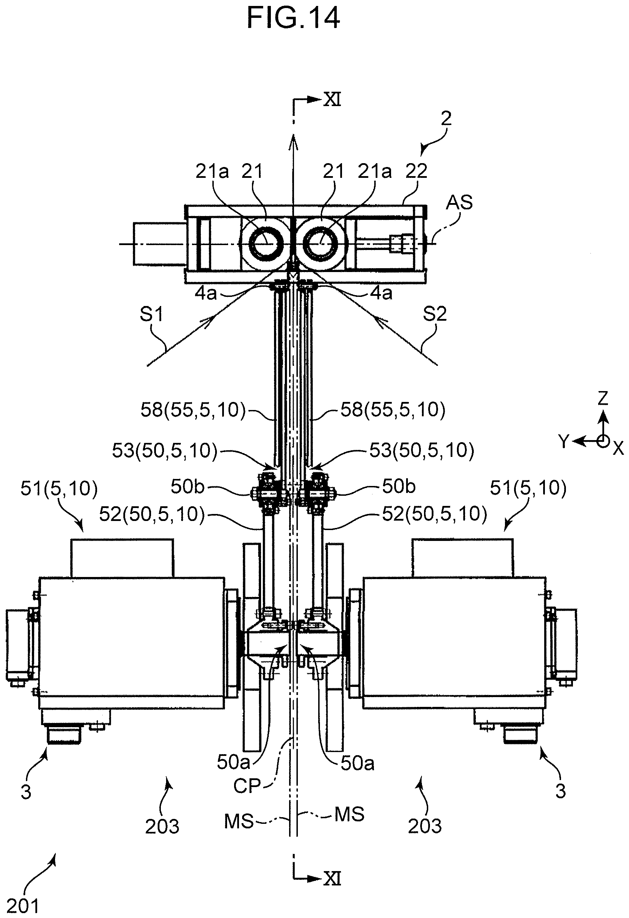

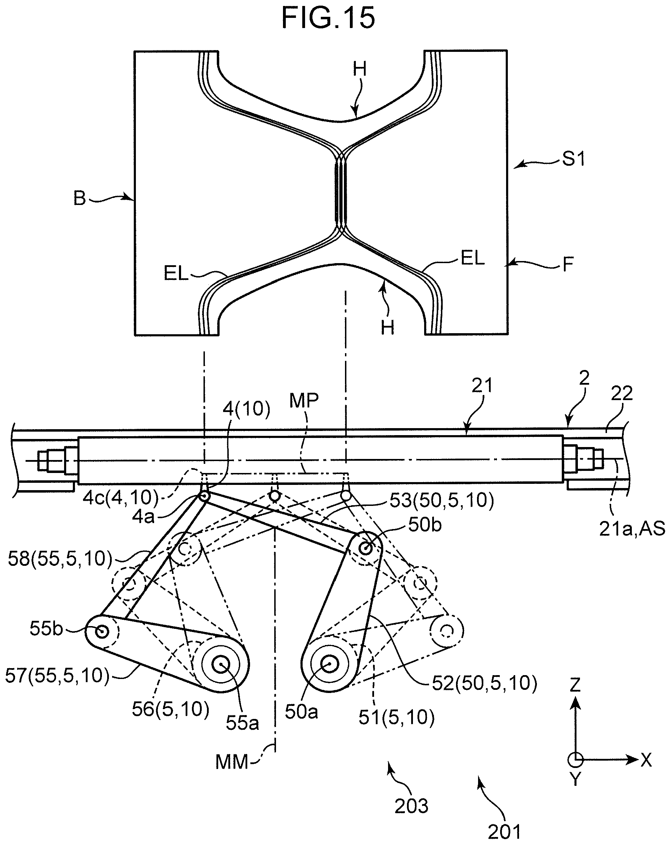

If the attitude of the guiding part of the present invention can be always maintained in the predetermined direction, or if the attitude of the guiding part does not greatly affect the trajectory of the elastic member even if the attitude of the guiding part changes, the plurality of link members can be omitted as in an elastic member arrangement part 203 of a manufacturing device 201 illustrated in FIGS. 14 and 15.

Specifically, in the pair of elastic member arrangement parts 203, unlike the elastic member arrangement part 3 illustrated in FIGS. 2 and 3, the plurality of link members 6a to 6d are omitted. Note that an interval for providing the plurality of link members 6a to 6d is unnecessary between the pair of elastic member arrangement parts 3, the pair of elastic member arrangement parts 3 is arranged so as to be close to each other.

### (E) Guiding Part

In the above embodiment, in the feeding mechanism, the guiding part 4 and the moving mechanism 5 are configured by separate members, but the configuration of the feeding mechanism is not limited to this. For example, in the feeding mechanism of the present invention, the moving mechanism and the guiding part may be integrally configured. For example, the guiding part may be configured by a holding hole provided in a connection part between the first guiding part side arm and the second guiding side arm. The elastic member is inserted into this holding hole, and the elastic member is guided between the pair of sheets via this holding hole.

The guiding part of the present invention is not limited to the shape of the above embodiment as long as the guiding part has a shape capable of guiding the plurality of elastic members between the pair of sheets. For example, the guiding part may be a member not extending in the feeding direction and having a circular shape, an elliptical shape, a spherical shape, or the like rotatably attached to the guiding part rotation shaft.

### (F) Retraction Movement Control

In the present invention, at least the reciprocation control may be executed, and the retraction movement control may be omitted.

### (G) Number of Elastic Members

In the manufacturing device of the present invention, the plurality of elastic members are not limited to be arranged on the pair of sheets, and only one elastic member may be arranged.

Note that the above-described specific embodiment mainly includes the invention having the following configuration.

A manufacturing device for an elastic laminate for solving the above problems provides a device for manufacturing an elastic laminate including a pair of sheets extending in a longitudinal direction and an elastic member nipped between the pair of sheets, the manufacturing device for an elastic laminate, including: a pair of nip rolls arranged in a state where each axis is orthogonal to the longitudinal direction so as to convey the pair of sheets and the elastic member in a conveying direction parallel to the longitudinal direction while nipping the pair of sheets in a state where the elastic member is interposed between the pair of sheets; a feeding mechanism including a guiding part that is provided upstream of the pair of nip rolls in the conveying direction and guides the elastic member between the pair of sheets while feeding the elastic member such that a longitudinal direction of the elastic member is along a feeding direction orthogonal to the axis, and a moving mechanism capable of reciprocating the guiding part in a moving direction parallel to the axis; and a reciprocation control part that controls the moving mechanism so that the guiding part reciprocates along a moving path extending in the moving direction, in which the moving mechanism includes a first rotation member rotatable about a first rotation center shaft orthogonal to a moving path plane including the moving path at a position away from the guiding part in the moving path plane and configured such that a first rotation radius connecting the guiding part and the first rotation center shaft is changeable, a first rotation drive part that rotates the first rotation member about the first rotation center shaft, a second rotation member rotatable about a second rotation center shaft parallel to the first rotation center shaft at a position away from the guiding part in the moving path plane, connected to the first rotation member so as to be rotatable each other, and configured such that a second rotation radius connecting the guiding part and the second rotation center shaft is changeable, and a second rotation drive part that rotates the second rotation member around the second rotation center shaft, and the reciprocation control part performs reciprocation control of synchronizing rotation of the first rotation member by the first rotation drive part and rotation of the second rotation member by the second rotation drive part so as to reciprocate the guiding part along the moving path.

A manufacturing method for an elastic laminate for solving the above problems provides a method for manufacturing an elastic laminate using the manufacturing device for an elastic laminate, the manufacturing method for an elastic laminate, including: a process of preparing the manufacturing device for the elastic laminate; a process of conveying the pair of sheets in the conveying direction so as to be guided between the pair of nip rolls; a process of synchronizing rotation of the first rotation member by the first rotation drive part and rotation of the second rotation member by the second rotation drive part so as to reciprocate the guiding part along the moving path; and a process of guiding the elastic member between the pair of sheets while feeding the elastic member in a longitudinal direction of the elastic member using the guiding part such that the elastic member is nipped between the pair of sheets by the pair of nip rolls.

According to the above, the guiding part can be reciprocated along the moving path without using a restriction mechanism accompanied by sliding friction of the slider with respect to the rail. This can reduce maintenance frequency. Specifically, in a state where the first rotation member and the second rotation member are rotatably connected to each other, the moving mechanism is controlled so that rotation by the first rotation drive part and rotation by the second rotation drive part are synchronized so as to move the guiding part along the moving path, whereby the guiding part can be reciprocated along the moving path with a change in the first rotation radius of the first rotation member and a change in the second rotation radius of the second rotation member. This can omit the restriction mechanism, and can reduce the frequency of maintenance due to existence of the restriction mechanism.

Since sliding resistance of the moving mechanism at the time of reciprocating the guiding part is small as compared with a case where the guiding part reciprocates by the belt bridged on the pair of pulleys reciprocating so as to repeat reversal of the moving direction as conventionally, the moving speed of the guiding part can be increased, and the manufacturing speed of the elastic laminate can be improved.

Note that connection between the first rotation member and the second rotation member may be a direct connection or an indirect connection via a separate interposing member.

In the manufacturing device for an elastic laminate, it is preferable that the guiding part has a shape extending in a predetermined direction, and a tip end part in the predetermined direction of the guiding part is provided with a holding part feedably holding the elastic member, and the manufacturing device further includes an attitude maintaining mechanism that maintains an attitude of the guiding part such that the holding part is positioned closer to the nip roll than the moving mechanism.

According to the manufacturing device described above, the guiding part can be always maintained at a position closer to the nip roll than the moving mechanism.

In the manufacturing device for an elastic laminate, it is preferable that the first rotation member includes a first driven arm connected to the first rotation drive part so as to rotate about the first rotation center shaft, and a first guiding part side arm connected rotatably about a first intermediate shaft parallel to the first rotation center shaft with respect to a part away from the first rotation center shaft in the first driven arm, the second rotation member includes a second driven arm connected to the second rotation drive part so as to rotate about the second rotation center shaft, and a second guiding part side arm connected rotatably about a second intermediate shaft parallel to the first rotation center shaft with respect to a part away from the second rotation center shaft in the second driven arm, and a part away from the first intermediate shaft in the first guiding part side arm and a part away from the second intermediate shaft in the second guiding part side arm are connected rotatably about a guiding part rotation shaft parallel to both the intermediate shafts, and the guiding part is provided rotatably about the guiding part rotation shaft with respect to the first guiding part side arm and the second guiding part side arm.

According to the manufacturing device described above, the guiding part can be moved along the moving path by relative rotation of the first driven arm and the first guiding part side arm and relative rotation of the second driven arm and the second guiding part side arm. Specifically, by the first driven arm and the second driven arm rotating in synchronization, the guiding part can be reciprocated along the moving path with relative rotation of the first guiding part side arm with respect to the first driven arm and relative rotation of the second guiding part side arm with respect to the second driven arm. In relative rotation of the first guiding part side arm with respect to the first driven arm, a first distance between the first rotation center shaft and the guiding part rotation shaft changes in the first rotation radius direction by changing an angle formed between the first driven arm and the first guiding part side arm. A second distance between the second rotation center shaft and the guiding part rotation shaft changes in the second rotation radius direction by relative rotation of the second guiding part side arm with respect to the second driven arm.

In the manufacturing device for an elastic laminate, it is preferable that the guiding part has a shape extending in a predetermined direction, and a tip end part in the predetermined direction of the guiding part is provided with a holding part feedably holding the elastic member, and the manufacturing device further includes a plurality of link members that, together with the first driven arm and the first guiding part side arm, constitute a parallel link mechanism for maintaining an attitude of the guiding part so that the holding part is positioned closer to the nip roll than the moving mechanism.

According to the manufacturing device described above, the guiding part can be always maintained at a position closer to the nip roll than the moving mechanism.

In the manufacturing device for an elastic laminate, it is preferable that the plurality of link members include a support member supporting the first rotation center shaft and a link rotation center shaft so as to restrict relative movement of the first rotation center shaft and the link rotation center shaft parallel to the first rotation center shaft, a driven link member supported by the link rotation center shaft in a state of being rotatable about the link rotation center shaft, and an intermediate link member and a guiding part side link member connected rotatably about a first intermediate link shaft parallel to the first rotation center shaft with respect to the driven link member, the intermediate link member is supported by the first intermediate shaft rotatably about the first intermediate shaft, the guiding part side link member is connected to the guiding part rotatably about a guiding part link shaft parallel to the first rotation center shaft, and a parallelogram is formed by a straight line connecting the guiding part rotation shaft and the first intermediate shaft, a straight line connecting the guiding part link shaft and the first intermediate link shaft, a straight line connecting the guiding part rotation shaft and the guiding part link shaft, and a straight line connecting the first intermediate shaft and the first intermediate link shaft, and a parallelogram is formed by a straight line connecting the first rotation center shaft and the first intermediate shaft, a straight line connecting the link rotation center shaft and the first intermediate link shaft, a straight line connecting the first rotation center shaft and the link rotation center shaft, and a straight line connecting the first intermediate shaft and the first intermediate link shaft.

According to the manufacturing device described above, the plurality of link members enable the guiding part to move along the moving path in a state of maintaining the attitude. A specific explanation is as follows. In the parallel link mechanism where two parallelograms are defined, the first rotation center shaft and the link rotation center shaft are supported by the support member in a state where relative movement is restricted. Due to this, the attitude of the guiding part during the movement period of the guiding part by the moving mechanism, that is, the orientation of the line connecting the guiding part rotation shaft and the guiding part link shaft is maintained in a state of matching the orientation of a line connecting the first rotation center shaft and the link rotation center shaft. That is, the guiding part can be moved along the moving path while maintaining the attitude corresponding to the orientations of these straight lines.

In the manufacturing device for an elastic laminate, it is preferable that a distance from the first rotation center shaft to the first intermediate shaft and a distance from the second rotation center shaft to the second intermediate shaft are identical, and a distance from the first intermediate shaft to the guiding part rotation shaft and a distance from the second intermediate shaft to the guiding part rotation shaft are identical.

According to the manufacturing device described above, since the rotation radius of the first driven arm and the rotation radius of the second driven arm are the same, and the rotation radius of the first guiding part side arm and the rotation radius of the second guiding part side arm are the same, when the moving path is set such that the center position of the moving path is positioned on a straight line passing through the center position in a direction along the axis between the first rotation center shaft and the second rotation center shaft, operations of the first rotation drive part and the second rotation drive part can be set symmetrically with reference to the center position, and hence it is possible to simplify calculation processing when rotation of the first rotation drive part and rotation of the second rotation drive part are synchronized with each other.

It is preferable that the manufacturing device for an elastic laminate further includes a retraction movement control part that performs retraction movement control of synchronizing rotation of the first rotation member and rotation of the second rotation member so as to retract the guiding part from the pair of nip rolls along the feeding direction in a period other than a period in which the reciprocation control is performed by the reciprocation control part.

According to the manufacturing device described above, by retracting the guiding part from the pair of nip rolls, an interval between the guiding part and the pair of nip rolls can be increased. Maintenance of the feeding mechanism can be performed using the interval.

In the manufacturing device for an elastic laminate, it is preferable that the moving path is set such that a distance in the feeding direction between a nip point, which is a position at which the elastic member is nipped between the pair of sheets by the pair of nip rolls, and the moving path becomes constant over an entirety of the moving path.

When the moving path of the guiding part with respect to an arrangement path of the elastic member is set, the moving path needs to be set so as to move the guiding part excessively according to the distance from the guiding part to the nip point. That is, a time difference corresponding to the distance between the guiding part and the nip point is generated between when a predetermined part of the elastic member is fed from the guiding part and when the predetermined part actually reaches the nip point. Therefore, in order to arrange the elastic member at a desired nip point, it is necessary to move the guiding part in the moving direction in advance in consideration of the time difference. Here, when the distance between the nip point and the moving path changes, the time difference varies depending on the position of the guiding part, and thus complicated calculation is required for setting or changing the moving path. According to the manufacturing device described above, since the distance in the feeding direction between the nip point and the moving path is constant over the entire moving path, the time difference becomes constant, and calculation for setting the moving path is easy.

In the manufacturing device for an elastic laminate, it is preferable that the moving path is a straight line parallel to the axis.

## Claims

1. device for manufacturing an elastic laminate including a pair of sheets extending in a longitudinal direction and an elastic member nipped between the pair of sheets, the manufacturing device for an elastic laminate, comprising:
a pair of nip rolls arranged in a state where each axis is orthogonal to the longitudinal direction so as to convey the pair of sheets and the elastic member in a conveying direction parallel to the longitudinal direction while nipping the pair of sheets in a state where the elastic member is interposed between the pair of sheets;
a feeding mechanism including a guiding part that is provided upstream of the pair of nip rolls in the conveying direction and guides the elastic member between the pair of sheets while feeding the elastic member such that a longitudinal direction of the elastic member is along a feeding direction orthogonal to the axis, and a moving mechanism capable of reciprocating the guiding part in a moving direction parallel to the axis; and
a reciprocation control part that controls the moving mechanism so that the guiding part reciprocates along a moving path extending in the moving direction,
wherein the moving mechanism includes a first rotation member rotatable about a first rotation center shaft orthogonal to a moving path plane including the moving path at a position away from the guiding part in the moving path plane and configured such that a first rotation radius connecting the guiding part and the first rotation center shaft is changeable, a first rotation drive part that rotates the first rotation member about the first rotation center shaft, a second rotation member rotatable about a second rotation center shaft parallel to the first rotation center shaft at a position away from the guiding part in the moving path plane, connected to the first rotation member so as to be rotatable each other, and configured such that a second rotation radius connecting the guiding part and the second rotation center shaft is changeable, and a second rotation drive part that rotates the second rotation member around the second rotation center shaft, and
the reciprocation control part performs reciprocation control of synchronizing rotation of the first rotation member by the first rotation drive part and rotation of the second rotation member by the second rotation drive part so as to reciprocate the guiding part along the moving path.

2. The manufacturing device for an elastic laminate according to claim 1, wherein
the guiding part has a shape extending in a predetermined direction, and a tip end part in the predetermined direction of the guiding part is provided with a holding part feedably holding the elastic member, and
the manufacturing device further includes an attitude maintaining mechanism that maintains an attitude of the guiding part such that the holding part is positioned closer to the nip roll than the moving mechanism.

3. The manufacturing device for an elastic laminate according to claim 1, wherein
the first rotation member includes a first driven arm connected to the first rotation drive part so as to rotate about the first rotation center shaft, and a first guiding part side arm connected rotatably about a first intermediate shaft parallel to the first rotation center shaft with respect to a part away from the first rotation center shaft in the first driven arm,
the second rotation member includes a second driven arm connected to the second rotation drive part so as to rotate about the second rotation center shaft, and a second guiding part side arm connected rotatably about a second intermediate shaft parallel to the first rotation center shaft with respect to a part away from the second rotation center shaft in the second driven arm, and
a part away from the first intermediate shaft in the first guiding part side arm and a part away from the second intermediate shaft in the second guiding part side arm are connected rotatably about a guiding part rotation shaft parallel to both the intermediate shafts, and the guiding part is provided rotatably about the guiding part rotation shaft with respect to the first guiding part side arm and the second guiding part side arm.

4. The manufacturing device for an elastic laminate according to claim 3, wherein
the guiding part has a shape extending in a predetermined direction, and a tip end part in the predetermined direction of the guiding part is provided with a holding part feedably holding the elastic member, and
the manufacturing device further includes a plurality of link members that, together with the first driven arm and the first guiding part side arm, constitute a parallel link mechanism for maintaining an attitude of the guiding part so that the holding part is positioned closer to the nip roll than the moving mechanism.

5. The manufacturing device for an elastic laminate according to claim 4, wherein
the plurality of link members include:
a support member supporting the first rotation center shaft and a link rotation center shaft so as to restrict relative movement of the first rotation center shaft and the link rotation center shaft parallel to the first rotation center shaft,
a driven link member supported by the link rotation center shaft in a state of being rotatable about the link rotation center shaft, and
an intermediate link member and a guiding part side link member connected rotatably about a first intermediate link shaft parallel to the first rotation center shaft with respect to the driven link member,
the intermediate link member is supported by the first intermediate shaft rotatably about the first intermediate shaft,
the guiding part side link member is connected to the guiding part rotatably about a guiding part link shaft parallel to the first rotation center shaft, and
a parallelogram is formed by a straight line connecting the guiding part rotation shaft and the first intermediate shaft, a straight line connecting the guiding part link shaft and the first intermediate link shaft, a straight line connecting the guiding part rotation shaft and the guiding part link shaft, and a straight line connecting the first intermediate shaft and the first intermediate link shaft, and a parallelogram is formed by a straight line connecting the first rotation center shaft and the first intermediate shaft, a straight line connecting the link rotation center shaft and the first intermediate link shaft, a straight line connecting the first rotation center shaft and the link rotation center shaft, and a straight line connecting the first intermediate shaft and the first intermediate link shaft.

6. The manufacturing device for an elastic laminate according to any one of claims 3 to 5, wherein a distance from the first rotation center shaft to the first intermediate shaft and a distance from the second rotation center shaft to the second intermediate shaft are identical, and a distance from the first intermediate shaft to the guiding part rotation shaft and a distance from the second intermediate shaft to the guiding part rotation shaft are identical.

7. The manufacturing device for an elastic laminate according to any one of claims 1 to 6, further comprising a retraction movement control part that performs retraction movement control of synchronizing rotation of the first rotation member and rotation of the second rotation member so as to retract the guiding part from the pair of nip rolls along the feeding direction in a period other than a period in which the reciprocation control is performed by the reciprocation control part.

8. The manufacturing device for an elastic laminate according to any one of claims 1 to 7, wherein the moving path is set such that a distance in the feeding direction between a nip point, which is a position at which the elastic member is nipped between the pair of sheets by the pair of nip rolls, and the moving path becomes constant over an entirety of the moving path.

9. The manufacturing device for an elastic laminate according to any one of claims 1 to 8, wherein the moving path is a straight line parallel to the axis.

10. A method for manufacturing an elastic laminate using the manufacturing device for an elastic laminate according to any one of claims 1 to 9, the manufacturing method for an elastic laminate, comprising:
a process of preparing the manufacturing device for the elastic laminate;
a process of conveying the pair of sheets in the conveying direction so as to be guided between the pair of nip rolls;
a process of synchronizing rotation of the first rotation member by the first rotation drive part and rotation of the second rotation member by the second rotation drive part so as to reciprocate the guiding part along the moving path; and
a process of guiding the elastic member between the pair of sheets while feeding the elastic member in a longitudinal direction of the elastic member using the guiding part such that the elastic member is nipped between the pair of sheets by the pair of nip rolls.
